# EUROPEAN PATENT APPLICATION

(11) **EP 4 759 279 A1**
(43) Date of publication of application: **17.06.2026**
(21) Application number: 24219015.5
(22) Date of filing: 11.12.2024
(51) Int. Cl.: A61F 7/02, A61F 13/01, A61F 13/02, A61F 7/03

(54) **KINESIOLOGY TAPE**

(71) Applicant: Haleon CH SARL, 1197 Prangins (CH)
(72) Inventor: The designation of the inventor has not yet been filed
(74) Representative: Haleon Patent Department

(57) **Abstract**

The present invention relates to elastic therapeutic tape, particularly kinesiology tape, for use in supporting muscles and joints of the body, for example, helping to ease pain from athletic and other physical injuries.

## Description

### FIELD OF THE INVENTION

The present invention relates to elastic therapeutic tape, particularly kinesiology tape, for use in supporting muscles and joints of the body, for example, helping to ease pain from athletic and other physical injuries. Particularly, the kinesiology tape comprises thermal cells and is capable of generating and applying heat.

### BACKGROUND TO THE INVENTION

Kinesiology tape may be used to support muscles, joints and/or connective tissues of the body. The tape is stretched and applied to a patient's body to provide a therapeutic benefit as a result of the recoil effect of the elasticity of the tape.

However, there remains a need for improvements that provide other therapeutic benefits.

### SUMMARY OF THE INVENTION

In a First Aspect of the Invention, there is provided a kinesiology tape comprising: a layer of elastic material having a first side and a second side opposite the first side, the layer of elastic material comprising a pressure sensitive adhesive disposed on the first side and a plurality of closely spaced heat cells, each of the heat cells being spaced apart from adjoining heat cells by spacer regions, each heat cell comprising an exothermic composition, wherein the exothermic composition is capable of generating heat upon contact with air.

In certain embodiments, the tape may comprise a second layer of elastic material having an inner surface connected to the spacer regions of the first layer, wherein the heat cells are formed by unconnected regions between the first and second layers and within which the exothermic composition is contained.

In certain embodiments, the tape may comprise a second layer of elastic material having an inner surface having a plurality of closely spaced indentations spaced apart from adjoining indentations by spacer regions connected to the first layer, wherein the heat cells are formed by the indentations within which the exothermic composition is contained.

In certain embodiments, the second side of the layer of elastic material comprises a plurality of closely spaced indentations spaced apart from adjoining indentations by spacer regions, the tape further comprising a second layer of elastic material connected to the spacer regions, wherein the heat cells are formed by the indentations within which the exothermic composition is contained.

In certain embodiments, the tape may comprise an intermediate layer of elastic material and a top layer of elastic material, the intermediate layer of elastic material comprising a plurality of closely spaced holes extending therethrough from an upper surface to a lower surface, wherein the lower surface is connected to the spacer regions of the first layer of elastic material thereby forming the heat cells and the top layer of elastic material is connected to the upper surface of the intermediate layer, sealing the heat cells and entrapping the exothermic composition.

In certain embodiments, the heat cells extend from the second side of the layer of elastic material and a continuous layer of elastic material is uniformly adhered to and overlays the heat cells, filling the spacer regions.

Particularly, at least one of the materials is gas permeable and the exothermic composition is capable of generating heat when a gas is received through the material.

More particularly, at least one of the materials is gas permeable and the exothermic composition is capable of generating heat when Oxygen is received through the material.

Yet more particularly, at least one of the materials has an oxygen permeability of at least 4.72 L/sec (10 ft³/min), preferably at least 9.44 L/sec (20 ft³/min), more preferably 9.44 L/sec (20 ft³/min) to 70.8 L/sec (150 ft³/min).

Particularly the elastic material(s) is/are elastic along a length thereof and inelastic along a width thereof.

Particularly, the layers of the kinesiology tape form a unitary, one-piece structure. More particularly, the layers of the kinesiology tape form a unitary, one-piece structure being coupled using an adhesive, interweaving, melting, chemical bonding, sewing, sonic welding or a combination thereof.

In preferred embodiments of the Invention, the heat cells have a substantially hexagonal prism shape.

Particularly, the pressure sensitive adhesive is covered with a peelable release liner Preferably, the kinesiology tape comprises a detachably mounted gas-impermeable cover.

In a Second Aspect of the Invention, there is provided a heat patch that comprises a body and a peelable release liner. The body comprises an upper gas permeable layer, a lower gas impermeable base layer and a pressure sensitive or heat activated adhesive layer that, in use, attaches the base layer to the skin of a patient's body. The heat patch comprises a plurality of closely spaced heat cells, each of the heat cells being spaced apart from adjoining heat cells by spacer regions. Preferably, the heat cells Preferably, each heat cell has the shape of a substantially hexagonal prism. The heat patch has the form of a bilaterally symmetrical butterfly shape, the outer contour of the patch being a smooth curve. In this way, the heat patch may conform to the contours of the human body and may be attached to the skin in different orientations.

Heat patches may further comprise at least one detachably mounted gas-permeable cover over the at least one oxygen permeable surface of the heat cells. Particularly, the at least one detachable mounted gas-permeable cover has an oxygen permeability level that is less than the oxygen permeability of the at least one oxygen permeable surface. The Inventors have discovered that the use of peelable layers with different rates or levels of oxygen permeability allows a user to control the heat of the patch. Limiting the rate of entry of oxygen through the oxygen permeable surface of the heat cells, lowers the rate at which the thermogenic reaction progresses resulting in lower heat output. Removing a peelable layer that has a lower level of oxygen permeability increase the rate of entry of oxygen through the oxygen permeable surface of the heat cells, increasing the rate at which the thermogenic reaction progresses resulting in higher heat output.

In embodiments of the First and Second Aspects, an insulating and/or reflective layer(s) may be provided, for example within the spacer regions and/or placed over the heat cells to direct heat/warming towards a patient's skin/body. Thus, the direction of heat is substantially uni-directional. The insulating and/or reflective layer(s) may be oxygen permeable.

### DESCRIPTION OF FIGURES

**FIGURE 1****:** Schematic of the general structure of the kinesiology tape and heat patch.

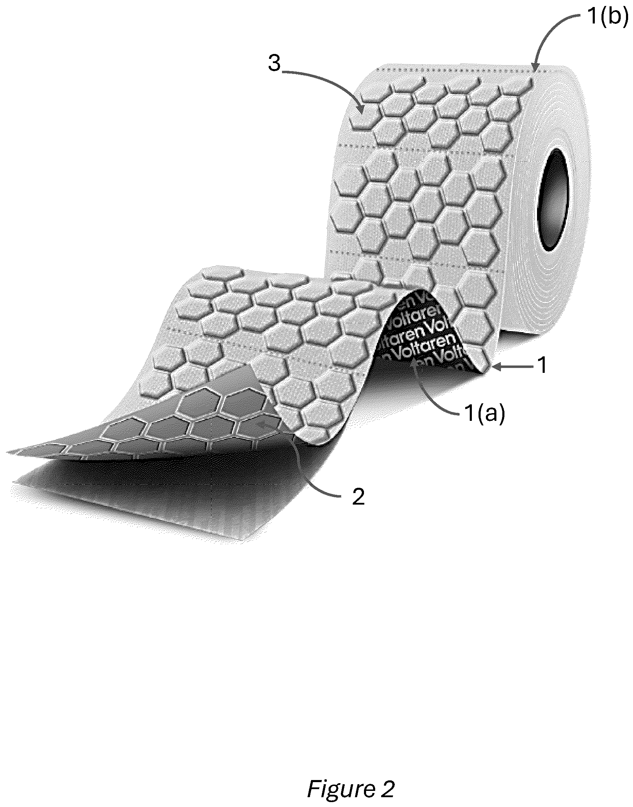

**FIGURE 2****:** Image of the finished kinesiology tape.

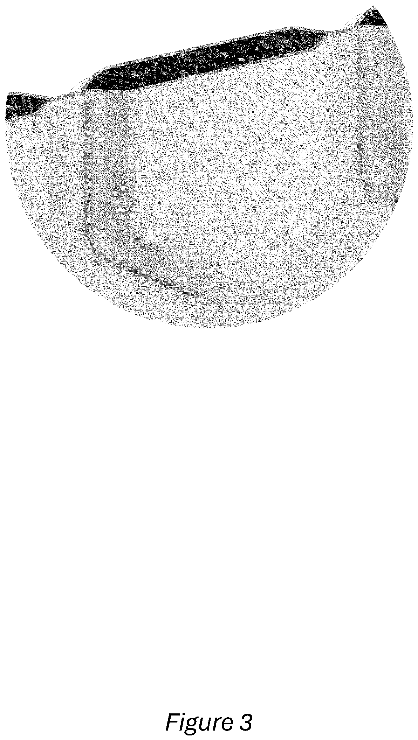

**FIGURE 3****:** Cross-section through a hexagonal heat cell showing the thermogenic material.

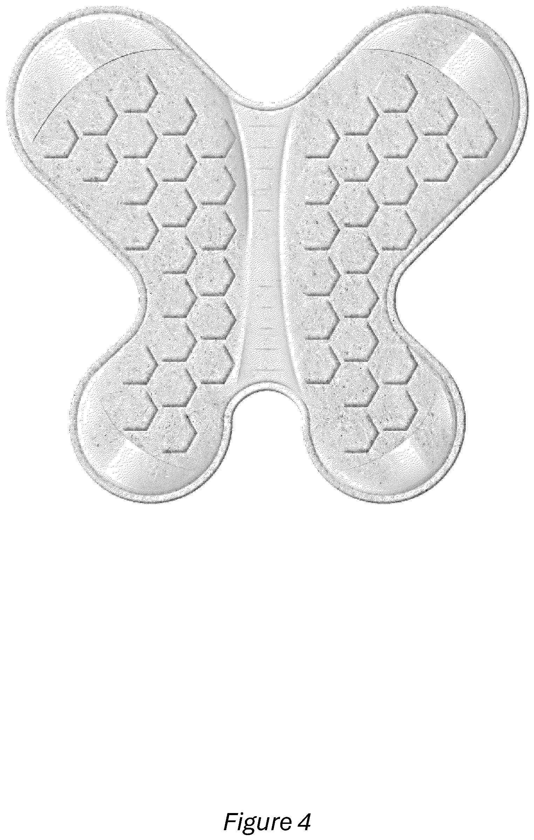

**FIGURE 4****:** Image of the butterfly-shaped heat patch.

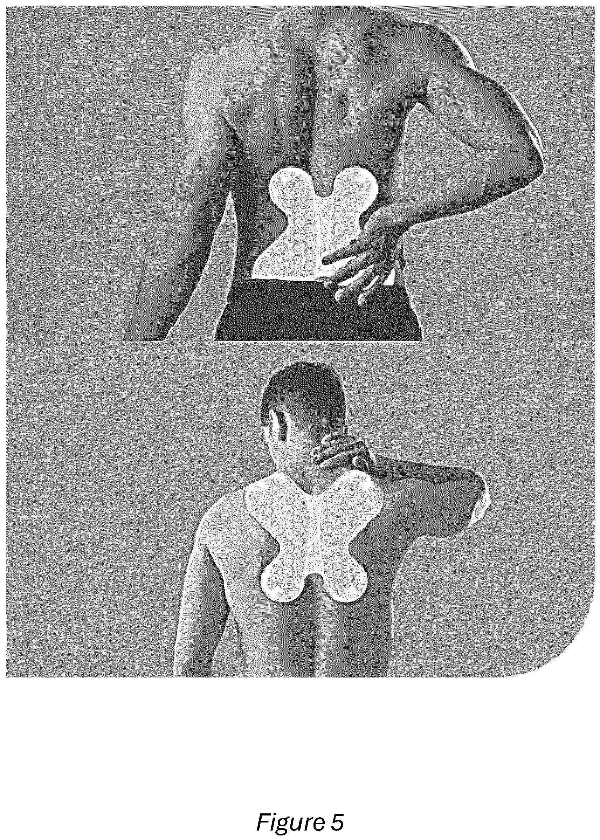

**FIGURE 5****:** Images showing reversible placement of the butterfly-shaped heat patch.

### DETAILED DESCRIPTION OF THE INVENTION

Thermotherapy, the application of heat to the body, may be used in a number of ways, for example to reduce pain and soreness and to increase blood flow. However, generally thermotherapy has to be applied using means other than kinesiology tape. The present inventors have developed improved kinesiology tapes comprising a plurality of closely spaced heat cells that contain an exothermic composition capable of generating heat upon contact with air. The kinesiology tape of the present invention avoids the need to use creams comprising menthol and methyl salicylate which can be greasy and messy and prevent tape from sticking. The kinesiology tapes are also thin and remain flexible throughout their use.

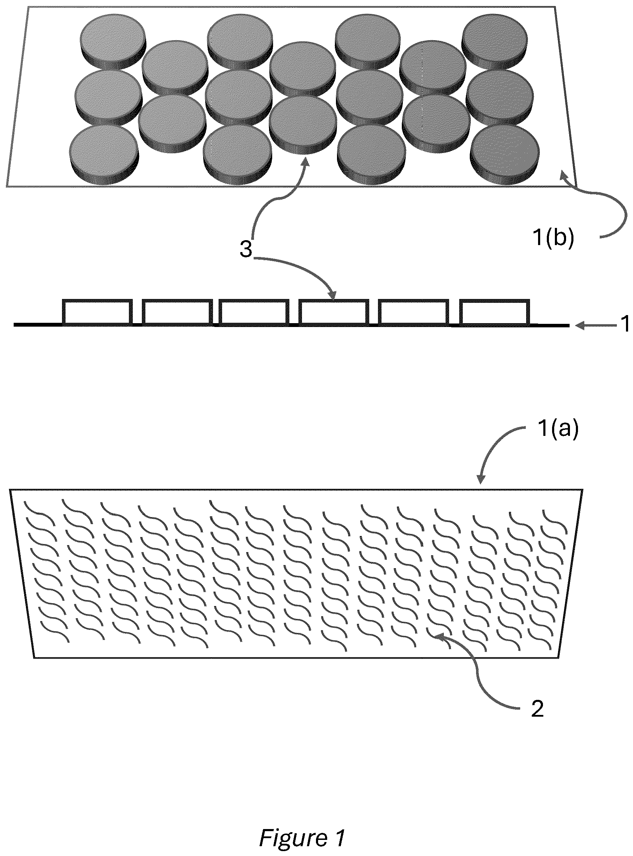

Referring to the features of the embodiment shown in Fig. 1, kinesiology tapes of the invention comprise a thin layer of elastic material (1) that has a first side (1a) and a second side (1b) opposite the first side. Generally, the first side comprises a pressure sensitive or heat activated adhesive (2) that, in use, attaches the tape to the skin of a patient's body. The adhesive may be water, particularly sweat, resistant. Particularly, prior to use, the pressure sensitive adhesive may be covered with a peelable release liner (not shown). The layer of elastic material is flexible and stretchable, particularly stretchable in one direction, usually along the long axis of the tape. "Elastic" means that the material will recover at least about 50 percent of its stretched length after the first pull. Elastic materials may comprise inelastic fibers such as cotton, wool, or synthetic material such as polyester or nylon. Suitable materials include woven and non-woven fabrics, for example comprising cotton or synthetic fibers, neoprene, rubber, latex, silicone and the like.

Kinesiology tape of the invention comprises a plurality of closely spaced heat cells (3). The heat cells are spaced apart from adjoining heat cells by spacer regions. The use of spacer regions ensures that the tape remains flexible and elastic. Each heat cell comprises an exothermic composition that is capable of generating heat upon contact with air. Preferably, the exothermic composition is a particulate exothermic composition, more particularly a particulate exothermic composition comprising iron powder, carbon and salt. Suitable compositions are disclosed in US5918590, incorporated herein by reference. In certain embodiments, the particulate exothermic composition comprises from about 30% to about 80% iron powder, from about 3% to about 25% carbon, from about 0.5% to about 10% of at least one metal salt, and from about 1% to about 40% water. In certain embodiments the carbon is activated carbon, non-activated carbon or a mixture thereof. Preferably at least one surface of the heat cells is oxygen permeable. The kinesiology tape may comprise a detachably mounted gas-impermeable cover over the at least one oxygen permeable surface of the heat cells (not shown). The cover prevents 'activation' of the exothermic composition during storage and prior to use.

The kinesiology tape may further comprise at least one detachably mounted gas-permeable cover over the at least one oxygen permeable surface of the heat cells. Particularly, the at least one detachable mounted gas-permeable cover has an oxygen permeability level that is less than the oxygen permeability of the at least one oxygen permeable surface. The Inventors have discovered that the use of peelable layers with different rates or levels of oxygen permeability allows a user to control the heat of the patch. Limiting the rate of entry of oxygen through the oxygen permeable surface of the heat cells, lowers the rate at which the thermogenic reaction progresses resulting in lower heat output. Removing a peelable layer that has a lower level of oxygen permeability increase the rate of entry of oxygen through the oxygen permeable surface of the heat cells, increasing the rate at which the thermogenic reaction progresses resulting in higher heat output.

Preferably, each heat cell has the shape of a hexagonal prism. The use of hexagonal shapes enables more efficient packing of the heat cells. In addition, the use of a tessellating hexagonal shape means that as the tape stretches, the heat cells can flex and deform in a linear direction.

The kinesiology tape is preferably manufactured in a substantially oxygen free environment, such as under a nitrogen atmosphere.

During manufacture, in one embodiment the layer of elastic material is formed to provide a plurality of discrete cells which are filled with an exothermic composition and then a second layer is used to seal the cells, thereby providing sealed heat cells in which the exothermic composition is entrapped.

In an alternative embodiment, a layer of gas permeable material is formed to provide a plurality of discrete cells which are filled with an exothermic composition. The layer of elastic material is used to seal the cells, thereby providing sealed heat cells in which the exothermic composition is entrapped. The gas permeable material may be cut or removed from the spacer regions.

In yet another embodiment, an elastic intermediate layer is used, for example a silicon layer. The intermediate layer comprises a plurality of closely spaced holes extending therethrough from an upper surface to a lower surface. The lower surface of the intermediate layer is connected to the spacer regions of the first layer of elastic material. In this manner, the heat cells are formed as wells that are then filled with an exothermic composition. A top layer of elastic material is then connected to the upper surface of the intermediate layer, sealing the heat cells and entrapping the exothermic composition.

### General

The term "comprising" encompasses "including" e.g. a composition "comprising" X may include something additional e.g. X + Y. Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP 5 2111.03. Thus, the term "consisting essentially of" when used in a claim of this invention is not intended to be interpreted to be equivalent to "comprising". The term "consisting of" and variations thereof means including and limited to (for example, the specific recited components or steps). The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein. The purpose of the above description is to illustrate some embodiments of the present invention without implying a limitation. It will be apparent to those skilled in the art that various modifications and variations may be made in the apparatus or procedure of the invention without departing from the scope or spirit of the invention.

## Claims

1. A kinesiology tape comprising:
a layer of elastic material having a first side and a second side opposite the first side, the layer of elastic material comprising a pressure sensitive adhesive disposed on the first side and a plurality of closely spaced heat cells, each of the heat cells being spaced apart from adjoining heat cells by spacer regions, each heat cell comprising an exothermic composition, wherein the exothermic composition is capable of generating heat upon contact with air.

2. The kinesiology tape of claim 1 which comprises a second layer of elastic material having an inner surface connected to the spacer regions of the first layer, wherein the heat cells are formed by unconnected regions between the first and second layers and within which the exothermic composition is contained.

3. The kinesiology tape of claim 1 which comprises a second layer of elastic material having an inner surface having a plurality of closely spaced indentations spaced apart from adjoining indentations by spacer regions connected to the first layer, wherein the heat cells are formed by the indentations within which the exothermic composition is contained.

4. The kinesiology tape of claim 1, wherein the second side of the layer of elastic material comprises a plurality of closely spaced indentations spaced apart from adjoining indentations by spacer regions, the tape further comprising a second layer of elastic material connected to the spacer regions, wherein the heat cells are formed by the indentations within which the exothermic composition is contained.

5. The kinesiology tape of claim 1 comprising an intermediate layer of elastic material and a top layer of elastic material, the intermediate layer of elastic material comprising a plurality of closely spaced holes extending therethrough from an upper surface to a lower surface, wherein the lower surface is connected to the spacer regions of the first layer of elastic material thereby forming the heat cells and the top layer of elastic material is connected to the upper surface of the intermediate layer, sealing the heat cells and entrapping the exothermic composition.

6. The kinesiology tape of claim 1, wherein the heat cells extend from the second side of the layer of elastic material and a continuous layer of elastic material is uniformly adhered to and overlays the heat cells, filling the spacer regions.

7. The kinesiology tape of any one of claims 1 to 6, wherein at least one of the materials is gas permeable and the exothermic composition is capable of generating heat when a gas is received through the material.

8. The kinesiology tape of claim 7, wherein the gas is oxygen.

9. The kinesiology tape of claim 8, wherein the at least one of the materials has an oxygen permeability of at least 4.72 L/sec (10 ft³/min), preferably at least 9.44 L/sec (20 ft³/min), more preferably 9.44 L/sec (20 ft³/min) to 70.8 L/sec (150 ft³/min).

10. The kinesiology tape of any preceding claim, wherein the elastic material(s) is/are elastic along a length thereof and inelastic along a width thereof.

11. The kinesiology tape of claim 2, 3, 4, 5 or 6, wherein the layers form a unitary, one-piece structure, being coupled using an adhesive, interwoven, melted, chemically bonded, sewn or sonically welded.

12. The kinesiology tape of any preceding claim, wherein the heat cells have a substantially hexagonal prism shape.

13. The kinesiology tape of claim 12, wherein the pressure sensitive adhesive is covered with a peelable release liner

14. The kinesiology tape of claim 12 or 13, comprising a detachably mounted gas-impermeable cover.
